# EUROPEAN PATENT SPECIFICATION

(11) **EP 0 874 657 B1**
(45) Date of publication and mention of the grant of the patent: **13.11.2002**
(21) Application number: 97900737.4
(22) Date of filing: 17.01.1997
(51) Int. Cl.: A61M 25/10

(54) **An isolated operating-room providing probe for the distal end of the oesophagus**
Eine einen isolierten Operationsraum für das distale Ende der Speiseröhre erzeugende Sonde
Système de champ opératoire isolé à sonde pour l'extrémité distale de l'oesophage

(30) Priority: 19.01.1996 IT CE960001
(43) Date of publication of application: 04.11.1998
(73) Proprietor: Paternuosto, Mario Immacolato, 81100 Caserta (IT)
(72) Inventor: Paternuosto, Mario Immacolato, 81100 Caserta (IT)
(74) Representative: Sarpi, Maurizio
(86) International application number: IT9700010
(87) International publication number: WO97026040

(56) References cited:
- WO-A-88/03389
- DE-A- 3 640 034
- DE-A- 3 915 289
- US-A- 4 022 216
- US-A- 5 314 409

## Description

The present invention relates to the surgery field and, more particularly, a probe which facilitates the execution, as an endoscopy, of a haemostasis (sclerosis or binding of varices) in case of bleeding.

Sclerosing or binding the bleeding varices of the oesophagus is an operating endoscopy which is executed on a hypovolaemic, often hyperammoniaemic patient markedly intolerant of movements which are not directed to a determined end or controllable either. An endoscopy should first locate the bleeding source and then carry out the operation as quickly as possible because of the precarious patient's conditions. However, the surgeon meets with a number of problems. First of all, administering a sedative to such patients is dangerous. In addition, there is a continuous reflux of blood from the gastric cavity to the operating area, represented by the distal end of the oesophagus, which is caused by the eructation and the continuous retching produced by air insufflation, water inlet and suction for clearing the operating area.
Under such conditions, both finding and treating the bleeding source need a lot of time.
Furthermore, both perivaricose and intravaricose techniques are difficult since:
a) in the perivaricose technique, the needle point cannot always be controlled when it softly pierces the oesophagus mucosa as the disappearance of the leading squirt of blood cannot be seen while the wheal is formed;
b) in the intravaricose technique, which may be wrongly carried out while being convinced that a perivaricose technique is executed, the injection of an excess of a sclerosing agent may have a serious consequence.

US-A-5314409 discloses an esophageal perfusion catheter having an outer housing, a plurality of flexible, tubular channel members within said housing, and two spaced balloon members encompassing said catheter. Said esophageal perfusion catheter was not designed neither for the emergency treatement of the bleeding varices of the oesophagus, nor for any other endoscopic therapeutic treatment. Further, the aspiration holes can easily be obstructed by the blood clots, in this way impeding the draining and the cleaning of the operating room defined by the inflated balloons in the oesophagus.

DE-A-3640034, which is used as a basis for the preamble of claim 1, envisages the introduction of an endoscope into a fenestrated tube sheath which is equipped with two balloons that form a chamber.
The endoscope carries out the sclerosis on the variceal piles which result to be procidentiae in the lumen of the tube itself and it allows suction of the haematic content from the stomach through said fenestration.
It is easy to observe that given the technical conditions in which the work should be carried out, it would indeed be rather cumbersome to carry out a ligature using the device described in said document, which was conceived when ligature was carried out using techniques which had not been sufficiently tested and refined.

According to the invention the above-mentioned problems and limits are overcome by providing a probe having the characteristics recited in claim 1, which may be operated by a different operator from that one operating the endoscopy and allows the operating area to be isolated from the gastric cavity and the proximal end of the oesophagus by inflating two pneumatic bladders. This allows the operation to be executed in several more favourable circumstances: easier cleaning of the operating area from the considerable quantity of blood through the suction channel which the device is provided with; blocking of the reflux of the blood from the stomach to the oesophagus; prevention of the reflux of the blood to the upper respiratory tract and reduction of ab ingestis pneumonias; more effective suction of the blood in the gastric cavity; and keeping of a desired pressure in the operating area.

Preferred embodiments of the probe are specified in the dependent claims.

It should be mentioned that the term proximal in the present description indicates the segment or the tract of the outer side of the device located closer to the mouth. In the same way the term distal will be used thereafter with the meaning that such a term has in anatomy, i.e. that tract, element or end located away from a conventional point of origin, that is the mouth in case of the alimentary canal.
The probe of the invention looks exteriorly like a tubular member of flexible material having a circular cross section and connecting, at its distal end, two pneumatic inflatable bladders at a suitable axial distance from each other, said bladders having different forms and larger diameters than the tubular member which moreover divides, at its proximal end, into two further tubular members of lower diameters.
The main tubular member is inside divided by partition walls into three channels, two of which having circular cross sections and communicating with a respective bladder which may be then inflated through the relative channel. The third channel is the central main tubular member embracing the two minor channels and passing through the bladders without communicating therewith.
In the length between the two bladders, the tubular member is provided with a plurality of through holes providing a direct communication between the operating area and the outside so as to prevent any increase in the pressure as a result of air insufflation into the two bladders.
The length of the tubular member after the proximal bladder is completely closed and carries a slidable coil provided with a small ring for hanging possible weights. Such a length ends at the proximal end outside the mouth of the patient where, as mentioned above, the tubular member divides into three tubular members. Actually, the two inner channels, through which air can be insufflated into the bladders, separate from the main tubular member outside the patient's mouth so as to form independent tubular members having circular cross section and lumen lower than the main tubular member. Thus, the intervention of side operators may be facilitated.

Further advantages and features of the probe of the present invention as well as the method of use will be more readily apparent from the following detailed description with reference to the accompanying drawings which show by way of a not limiting example a preferred embodiment. In the drawings:
Fig. 1 shows schematically the device or probe as a whole;
Fig. 2 is a front view in enlarged scale of the two pneumatic bladders and the length of the connecting tubular member;
Fig. 3 is a longitudinal section view of Fig. 2;
Fig. 4 is an enlarged detail of the length of tubular member about which the coil is wound;

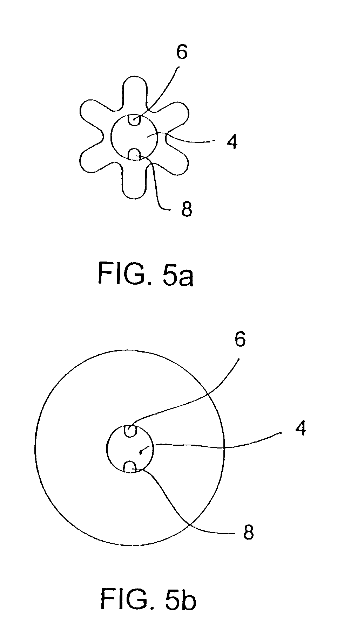

Figs. 5a and 5b are two sectioned views along the lines X-X' and Y-Y' of Fig. 2, respectively;
Fig. 6 shows schematically the probe used for providing an operating room in the oesophagus;
Fig. 7 shows schematically the probe used after the sclerosis for the suction of blood in the gastric cavity;
Fig. 8 shows schematically a further step of suction of blood, the distal pneumatic bladders being attached to pylorus.

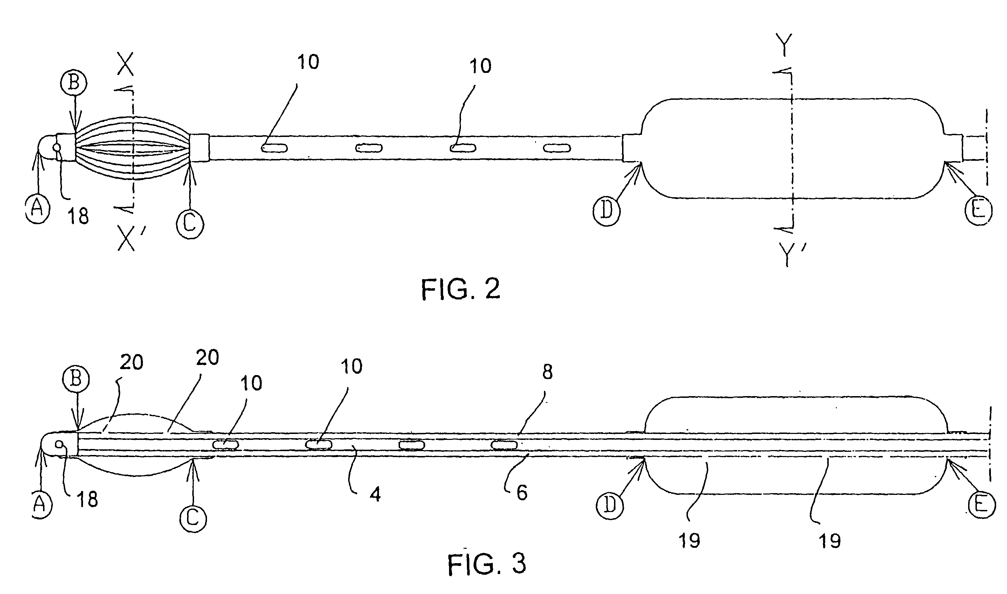

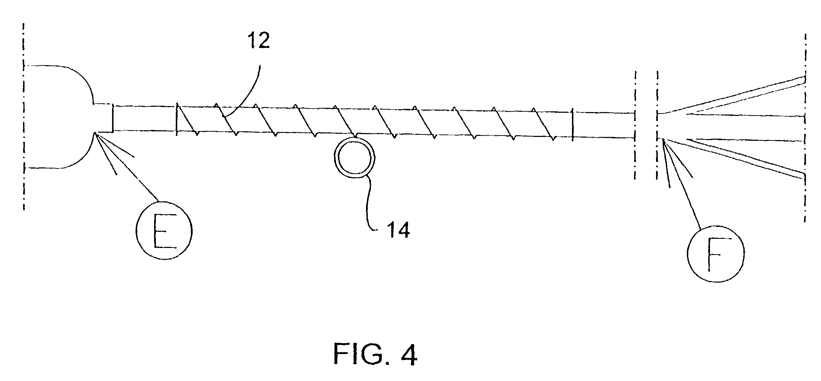

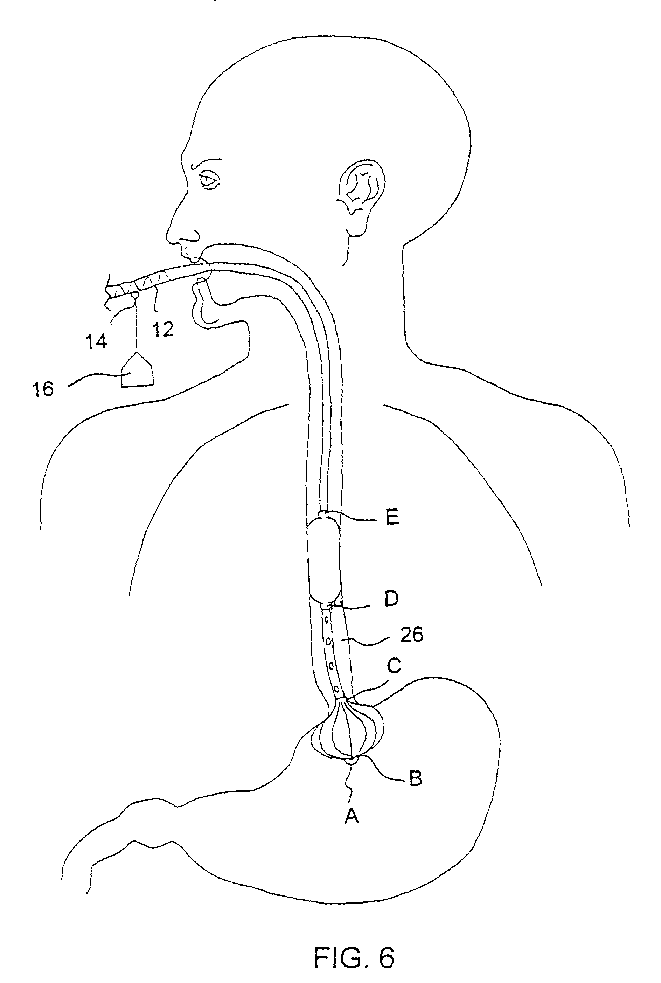

With reference to the figures, the device or probe of the invention consists of a composite, flexible tubular member which in a preferred embodiment has the length of 180 cm and is formed of a pipe A-H of circular section which divides at F into further two tubular members FG and FI having circular sections and reduced diameters.
Two pneumatic chambers or bladders BC and DE are located in the main tubular member between A and F. Bladder BC is of natural rubber and, by way of example, has a width of 1.5 cm, a length of 3 cm under deflated conditions and a six-radius-symmetry form or other forms. It should be noted that such dimensions as well as those indicated thereafter are not binding as they may be varied as a function of the operating conditions and the particular patient. Also bladder DE is of natural rubber and, by way of example, has a width of 2.5 cm, a length of 8 cm under deflated conditions and a circular section.
The length of the tubular member between the two bladders BC and DE is perforated. The holes 10 of ellipsoidal form are preferably in a number of eight. As can be seen from Figs. 2 and 3, the first two holes (major axis 7 mm, minor axis 3 mm) from bladder BC are diametrically opposed but offset to each other by the same length as the major axis. The other holes of the same dimensions are also diametrically opposed with parallel major axes and offset to one another by a length as high as the double of their major axis.
Advantageously, a first length of 5 cm from bladder BC of the tubular member between bladders BC and DE has a colour which is different from that of the remaining pipe.
A slidable coil 12 provided with a ring 14 for hanging a possible weight 16 allowing a fixed subcardias traction, as shown in Fig. 6, is provided along the tubular member between bladder DE and point F (Fig. 4).

The length between point A and bladder BC is provided with a crossing hole 18 of 2 mm diameter, ends in a round form and has a length of 1 cm (Fig. 2).

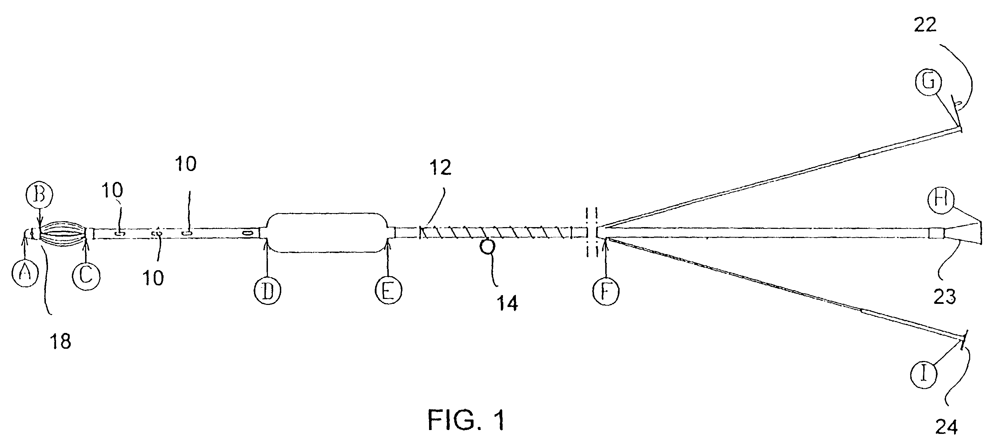

The inner structure of the probe is now described with reference to the longitudinal section of Fig. 3. The length of pipe between point A and point B has no lumen. The length of pipe from point B to point F has a wall thickness of 0.5 mm and an inner diameter of the larger main channel, indicated at 4 and including two further channels 6 and 8, of 3 mm. Channels 6 and 8 have a lumen of 1 mm and are located in parallel on either inner side of the main channel 4 at the same distance from the centre thereof. Preferably, channels 6 and 8 have a length which is different from that of channel 4. Channel 8 has the function of insufflating air into bladder DE through four holes 19 of 2 mm diameter. The other channel 6 has the function of insufflating air into bladder BC through two holes 20 of 2 mm diameter. The two channels 6 and 8 are closed at their initial ostium by plugs integral therewith 22, 24, respectively (Fig. 1).

The length F-H of the tubular member has at its proximal end (point H) a flared terminal 23.

According to a preferred embodiment the probe is made of thermoplastic material except for the bladders which are of natural rubber.

The method of use is now described with reference to the three following execution steps or times.

### 1) Oesophagus Time

After the introduction of the probe through the patient's mouth, the gastric cavity is isolated from the oesophagus tract by inflating the distal bladder BC. Thereafter the operator introduces the endoscopy into the operating area by slipping it along the side of the proximal, still deflated bladder DE. Thus, upon inflating with air the latter bladder, the proximal tract of the oesophagus is isolated from the operating area 26 where the endoscopy carries out the sclerosis (Fig. 6). Operating area 26 communicates with the outside through the holes 10 of the main tubular member 4, which prevents any increase in the pressure caused by air insufflation into the probe. The same holes 10 are also used for continuously cleaning the operating area: any liquid inlet may either be sucked through such holes 10 or through the channel of the endoscopy (not shown in the figures).

The advantages of the method according to the invention are as follows:
a) neither air nor water inlet into the stomach so as to reduce the risk of reflux which is, however, made impossible by the bladder closing the cardias, as shown in Fig. 6;
b) prevention of an ingestis pneumonia, which is a very serious trouble in such patients, by the presence of the proximal bladder in the oesophagus so as to form a cover for the operating area (Fig. 6). It should be mentioned that such an event is rather frequent as the reflection of the cough is turbid: the patient is wasted and hyperammoniaemic and, in most cases, has taken a sedative;
c) compression of the varices in two points so as to reduce the risk of a pulmonary embolus. Such a circumstance is greatly appreciated when the actuation of a perivaricose technique, upon eradicating, is very difficult because of fibrosis due to preceding interventions. Under such conditions, the wall of the oesophagus may be sclerosed with the risk of possible complications if the perivaricose technique is carried out repeatedly;
d) elimination of the problem due to the conveyance of the diluted blood towards pylorus due to the washing of the operating area with water, thus avoiding the risk of coma;
e) considerable facilitation of the binding technique when the varices are being broken, which is very difficult because of the considerable quantity of blood limiting the field of sight already limited by the binding equipment, since in case of emergency sclerosis or binding the serious problem of cleaning the operating area is overcome in a very short time. This is due to the fact that cleaning and washing are devolved on another operator acting in cooperation with but independently of the first operator who manages the endoscopy;
f) continuous presence of the operating needle in the operating channel in case of emergency sclerosis as the cleaning and the suction are carried out by the probe of the invention.

### 2) Gastric Time

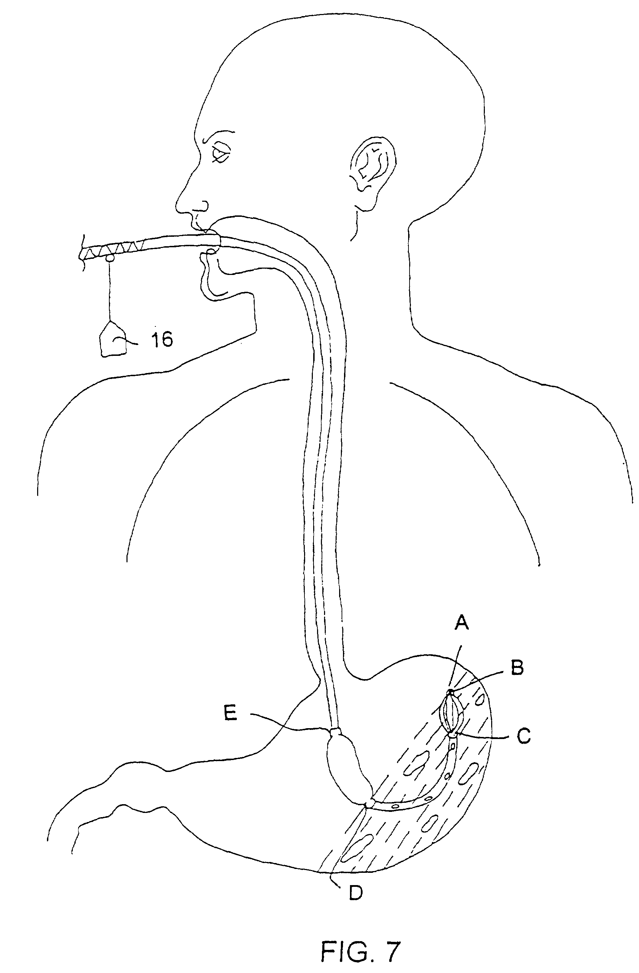

After the sclerosis, the probe is removed by deflating the two bladders and its slotted part is brought to the best possible position for the suction of blood by means of a polypus loop or biopsy pliers, as shown in Fig. 7. However, some absolutely not removable coagulation generally remains so that the third step, i.e. the Duodenal Time, is required.

### 3) Duodenal Time

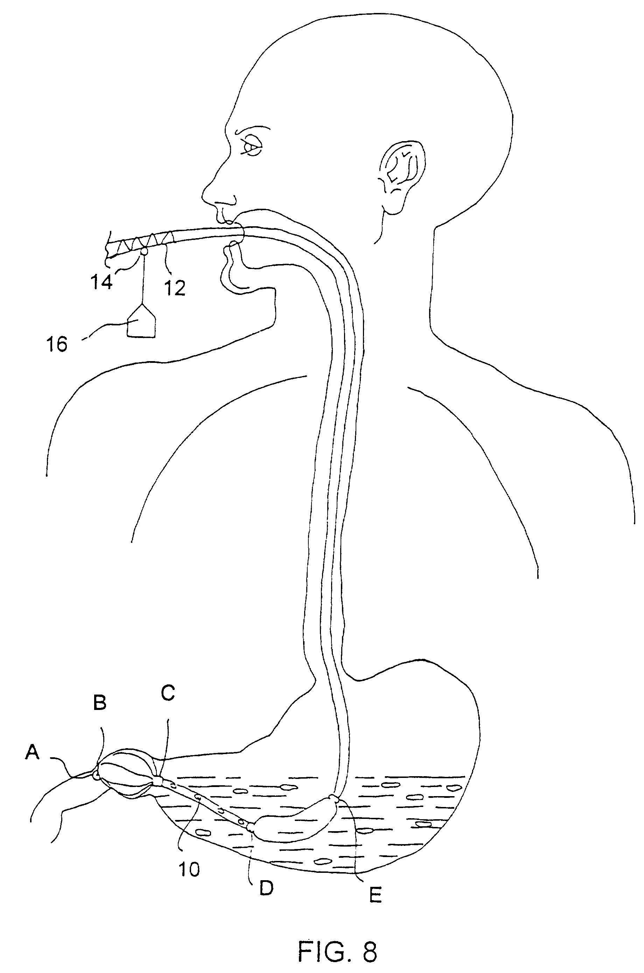

The distal bladder is positioned (Fig. 8) in the bulb by means of biopsy pliers or a loop engaging the towing cable of the probe and then it is attached to pylorus by a suitable insufflation. This is the only way to remove at best blood and coagulation because of the strategic position of the distal bladder in the bulb and the provision of the holes allowing washing and suction during the 24 hours.

## Claims

1. A probe which facilitates the execution of haemostasis techniques in case of bleeding varices of the oesophagus formed of a composite, flexible device of tubular form crosswise divided into three channels (4, 6, 8) and provided at its distal end with two bladders (BC, DE) placed at an axial distance from each other and inflatable independently of each other by air insufflated through two respective channels (6, 8) so that, upon inflating said two bladders (BC, DE), it is possible to isolate the axial length between the two bladders from the gastric cavity and the proximal tract of the oesophagus, thus forming an isolated operating room (26) at the distal end of the oesophagus, said operating room (26) being always kept in communication with the outside through the channel (4) which is not connected to said bladders (BC, DE); one of said three channels (4, 6, 8) having a larger diameter and including the other two channels of like lower diameters, said channel (4) of larger diameter being provided with through holes (10) in the length between said bladders (BC, DE) for establishing a direct communication between the probe and the outside, each channel (6, 8) of lower diameter being connected to the respective one of the two bladders (BC, DE), **characterized in that** the proximal bladder (DE) has a length of 8 cm and a circular section of 2.5 cm diameter under deflated conditions, while the distal bladder (BC) has a length of 3 cm and a diameter of 1.5 cm under deflated conditions; the holes (10) of the channel of larger diameter (4) positioned between the two bladders (BC, DE) are in a number of eight, have an ellipsoidal form with major axis parallel to the axis of the channel, and are placed in four pairs in diametrically opposed positions along the periphery of the larger channel apart from the channels of lower diameter; the channels of lower diameters communicate with the respective bladders (BC,DE) through holes (19,20), which are in a number of four in the proximal bladder (DE) and in a number of two in the distal bladder (BC), said holes (19, 20) being circular with a diameter of 2 mm and being located at a distance of 14 mm from one another in series along only one part of the periphery of the relative channel (6, 8).

2. The probe of claim 1, wherein it has a length of about 180 cm, a wall thickness of 0.5 mm, an inner diameter of the larger channel (4) of 3 mm, an inner diameter of the lower channels (6, 8) of 1 mm.

3. The probe of claim 1, wherein said tubular structure is of any thermoplastic material except for the bladders (BC, DE) which are of natural rubber.

4. The probe of claim 1, wherein the first two holes (10) closer to the distal bladder (BC) are diametrically opposed and offset to each other by the same length as their major axis, while the remaining holes (10) are offset to one another by a length as high as the double of their major axis.

5. The probe of claims 1 and 4, wherein the length of the major axis of said holes (10) is 7 mm and that of the minor axis is 3 mm.

6. The probe of claim 1, wherein the channels (6, 8) of lower diameters forme tracts passing within said bladders (BC, DE) wherby said circular holes (19,20) are arranged in the respective tracts.

7. The probe of claim 1, wherein one (BC) of the two bladders is located at the distal end of the probe and has dimensions lower than those of the other bladder (DE), that is located at a proximal position with respect to the former and has elongated form and circular section.

8. The probe of claims 1 and 7, wherein the distances of the proximal end and the distal end of the longer bladder (DE) from the end of the probe are 23.9 cm and 16 cm, respectively, while the distances of the proximal end and the distal end of the shorter bladder (BC) from the end of the probe are 4 cm and 1 cm, respectively, and the distance between the two bladders (BC, DE) is 12 cm.

9. The probe of claim 1, wherein the two lower channels (6, 8) are divided from the larger channel (4) at the proximal end of the probe by a length of 25 cm and are provided each with a plug (22, 24) integral therewith.

10. The probe of claim 1, wherein the larger channel (4) is flared (23) at its proximal end (H).

11. The probe of claim 1, wherein the portion of the probe with the through holes (10) closer to the distal bladder (BC) has a different colour by a length of some centimetres.

12. The probe of claim 1, wherein the section of the larger channel (4) from the distal bladder (BC) to the end point (A) of the probe is solid, has a length of 1 cm and is provided with a through hole (18) of a few millimetre diameter.

13. The probe of claim 1, wherein it is provided with a coil (12) which is wound about a length (FE) of the probe and provided with a ring (14) to which a weight (16) allowing a fixed subcardias traction may be hung.

14. The probe of claim 1, wherein the proximal bladder (DE) under deflated conditions is such as to allow the operator to slip an endoscope to the operating area.

## Patentansprüche

1. Sonde zur leichteren Durchführung von Hämostasetechniken bei Varizenblutungen im Ösophagus, bestehend aus einer zusammengesetzten, flexiblen Vorrichtung röhrenförmiger Gestalt, die in Querrichtung in drei Kanäle (4, 6, 8) unterteilt und an ihrem distalen Ende mit zwei Blasen (BC, DE) versehen ist, die im axialen Abstand voneinander angeordnet und mittels Luft, die durch zwei Kanäle (6, 8) eingeblasen wird, unabhängig voneinander aufblasbar sind, so dass es beim Aufblasen der beiden Blasen (BC, DE) möglich ist, die axiale Länge zwischen den beiden Blasen von der Magenhöhle und dem proximalen Trakt das Ösophagus zu isolieren und so einen isolierten Operationsraum (26) am distalen Ende des Ösophagus zu bilden, wobei der Operationsraum (26) über den Kanal (4), der nicht mit den Blasen (BC, DE) verbunden ist, stets mit der Außenseite in Verbindung bleibt, wobei einer der drei Kanäle (4, 6, 8) einen größeren Durchmesser aufweist und die anderen beiden Kanäle mit den entsprechend kleineren Durchmessern enthält, wobei der Kanal (4) mit dem größeren Durchmesser mit durchgehenden Löchern (10) in der Länge zwischen den Blasen (BC, DE) zum Herstellen einer direkten Verbindung zwischen der Sonde und der Außenseite versehen ist, wobei jeder Kanal (6, 8) mit kleinerem Durchmesser mit einer der beiden Blasen (BC, DE) verbunden ist, **dadurch gekennzeichnet, dass** die proximale Blase (DE) eine Länge von 8 cm und einen kreisförmigen Abschnitt mit einem Durchmesser von 2,5 cm unter Deflationsbedingungen aufweist, während die distale Blase (BC) eine Länge von 3 cm und einen Durchmesser von 1,5 cm unter Deflationsbedingungen aufweist, die zwischen den beiden Blasen (BC, DE) angeordneten Löcher (10) im Kanal mit dem größeren Durchmesser (4) in einer Anzahl von acht Löchern vorliegen, eine ellipsoide Form mit der Hauptachse parallel zur Achse des Kanals aufweisen und in vier Paaren sich diametral gegenüberliegend auf dem Umfang des größeren Kanals im Abstand von den Kanälen mit kleinerem Durchmesser angeordnet sind, die Kanäle mit dem kleineren Durchmesser mit den jeweiligen Blasen (BC, DE) über Löcher (19, 20) in Verbindung stehen, die in einer Anzahl von vier Löchern in der proximalen Blase (DE) und in einer Anzahl von zwei Löchern in der distalen Blase (BC) angeordnet sind, wobei diese Löcher (19, 20) kreisförmig sind, einen Durchmesser von 2 mm aufweisen und im Abstand von 14 mm voneinander in Reihe entlang nur eines Teils des Umfangs des jeweiligen Kanals (6, 8) angeordnet sind.

2. Sonde nach Anspruch 1, worin die Sonde eine Länge von ca. 180 cm, eine Wanddicke von 0,5 mm, einen Innendurchmesser des größeren Kanals (4) von 3 mm und einen Innendurchmesser der kleineren Kanäle (6, 8) von 1 mm aufweist.

3. Sonde nach Anspruch 1, worin die röhrenförmige Konstruktion aus einem beliebigen thermoplastischen Material besteht, mit Ausnahme der Blasen (BC, DE), die aus Naturkautschuk bestehen.

4. Sonde nach Anspruch 1, worin die ersten, der distalen Blase (BC) näher liegenden, beiden Löcher (10) sich diametral gegenüberstehen und über die Länge ihrer Hauptachse gegeneinander versetzt sind, während die restlichen Löcher (10) über eine Länge, die doppelt so lang sein kann wie ihre Hauptachse, gegeneinander versetzt sind.

5. Sonde nach Anspruch 1 und 4, worin die Länge der Hauptachse der Löcher (10) 7 mm und die Länge der Nebenachse 3 mm betragen.

6. Sonde nach Anspruch 1, worin die Kanäle (6, 8) mit kleinerem Durchmesser innerhalb der Blasen (BC, DE) verlaufende Gänge bilden, wodurch die kreisförmigen Löcher (19, 20) in den jeweiligen Gängen angeordnet sind.

7. Sonde nach Anspruch 1, worin eine (BC) der beiden Blasen am distalen Ende der Sonde angeordnet ist und kleinere Abmessungen aufweist als die andere Blase (DE), die proximal relativ zur ersten Blase angeordnet ist und eine längliche Gestalt und einen kreisförmigen Querschnitt aufweist.

8. Sonde nach Anspruch 1 und 7, worin die Abstände des proximalen Endes und des distalen Endes der längeren Blase (DE) vom Ende der Sonde 23,9 cm bzw. 16 cm betragen, während die Abstände des proximalen Endes und des distalen Endes der kürzeren. Blase (BC) vom Ende der Sonde 4 cm bzw. 1 cm betragen, und der Abstand zwischen den beiden Blasen (BC, DE) 12 cm ist.

9. Sonde nach Anspruch 1, worin die beiden kleineren Kanäle (6, 8) vom größeren Kanal (4) am proximalen Ende der Sonde über eine Länge von 25 cm getrennt sind und jeweils mit einem einstückig damit geformten Pfropf (22, 24) versehen sind.

10. Sonde nach Anspruch 1, worin der größere Kanal (4) an seinem proximalen Ende (H) aufgeweitet ist (23).

11. Sonde nach Anspruch 1, worin der, der distalen Blase (BC) näher liegende Abschnitt der Sonde mit den durchgehenden Löchern (10) über einige zentimeter eine andere Farbe aufweist.

12. Sonde nach Anspruch 1, worin der Abschnitt des größeren Kanals (4) von der distalen Blase (BC) bis zum Endpunkt (A) der Sonde fest ist, eine Länge von 1 cm aufweist und mit einem durchgehenden Loch (18) mit einem Durchmesser von einigen wenigen Millimetern versehen ist.

13. Sonde nach Anspruch 1, worin die Sonde mit einer Spule (12), die über eine Länge (FE) der Sonde gewickelt ist, und mit einem Ring (14) versehen ist, an dem ein Gewicht (16) zum Aufhängen eines festen Subkardia-Zugs befestigt werden kann,

14. Sonde nach Anspruch 1, worin die proximale Blase (DE) es dem Chirurg unter Deflationsbedingungen gestattet, ein Endoskop zum Operationsraum zu schieben.

## Revendications

1. Sonde qui facilite l'exécution des techniques hémostatiques dans le cas de varices hémorragiques de l'oesophage, formée d'un dispositif flexible composite, de forme tubulaire, divisé dans le sens de la largeur en trois canaux (4, 6, 8) et pourvu à son extrémité distale de deux poches (BC, DE) placées à une distance axiale l'une de l'autre et gonflable indépendamment l'une de l'autre par insufflation d'air à travers deux canaux respectifs (6, 8) de telle sorte que, lors de l'insufflation desdites deux poches (BC, DE), il est possible d'isoler la longueur axiale entre les deux poches à partir de la cavité gastrique et du tractus proximal de l'oesophage, formant ainsi un champ opératoire isolé (26) à l'extrémité distale de l'oesophage, la communication entre ledit champ opératoire (26) et l'extérieur étant toujours maintenue à travers le canal (4) qui n'est pas connecté auxdites poches (BC, DE) ; l'un desdits trois canaux (4, 6, 8) ayant un diamètre supérieur et comprenant deux autres canaux de diamètres inférieurs égaux, ledit canal (4) de diamètre supérieur étant pourvu de trous débouchants (10) dans la longueur entre lesdites poches (BC, DE) pour établir une communication directe entre la sonde et l'extérieur, chaque canal (6, 8) de diamètre inférieur étant connecté à l'une respective des deux poches (BC, DE), **caractérisée en ce que** la poche proximale (DE) a une longueur de 8 cm et une section circulaire de 2,5 cm de diamètre en état dégonflé, alors que la poche distale (BC) a une longueur de 3 cm et un diamètre de 1,5 cm en état dégonflé, les trous (10) du canal de diamètre supérieur (4) positionné entre les deux poches (BC, DE) sont au nombre de huit, ont une forme ellipsoïdale avec le grand axe parallèle à l'axe du canal, et sont placés en quatre paires dans des positions diamétralement opposées le long de la périphérie du canal le plus grand à l'écart des canaux de diamètre inférieur; les canaux de diamètre inférieur communiquent avec les trous débouchants (19, 20) des poches respectives (BC, DE), trous qui sont au nombre de quatre dans la poche proximale (DE) et au nombre de deux dans la poche distale (BC), lesdits trous (19, 20) étant circulaires avec un diamètre de 2 mm et étant situés à une distance de 14 mm les uns des autres en série le long d'une seule partie de la périphérie du canal respectif (6, 8).

2. Sonde selon la revendication 1, dans laquelle elle a une longueur d'environ 180 cm, une épaisseur de paroi d'environ 0,5 mm, un diamètre interne du canal le plus grand (4) de 3 mm, un diamètre interne des canaux les plus petits (6, 8) de 1 mm.

3. Sonde selon la revendication 1, dans laquelle ladite structure tubulaire est fabriquée dans un matériau thermoplastique quelconque, excepté les poches (BC, DE) qui sont en caoutchouc naturel.

4. Sonde selon revendication 1, dans laquelle les deux premiers trous (10) les plus proches de la poche distaie (BC) sont diamétralement opposés et décalés les uns des autres de la même longueur que leur grand axe, alors que les trous restants (10) sont décalés les uns par rapport aux autres d'une longueur équivalente au double de leur grand axe.

5. Sonde selon les revendications 1 et 4, dans laquelle la longueur du grand axe desdits trous (10) est de 7 mm et celle du petit axe est de 3 mm.

6. Sonde selon la revendication 1, dans laquelle les canaux (6, 8) de diamètres inférieurs forment des conduits passant à l'intérieur desdites poches (BC, DE), moyennant quoi lesdits trous circulaires (19, 20) sont agencés dans les conduits respectifs.

7. Sonde selon la revendication 1, dans laquelle l'une (BC) des deux poches se situe à l'extrémité distale de la sonde et a des dimensions inférieures à celles de l'autre poche (DE), qui est située à une position proximale par rapport à la première et a une forme allongée et une section circulaire.

8. Sonde selon les revendications 1 et 7, dans laquelle l'extrémité proximale et l'extrémité distale de la poche la plus longue (DE) sont, à partir de l'extrémité de la sonde, distants respectivement de 23,9 cm et 16 cm, alors que l'extrémité proximale et de l'extrémité distale de la poche la plus courte (BC) sont, à partir de l'extrémité de la sonde, distants respectivement de 4 cm et de 1 cm, respectivement, et la distance entre les deux poches (BC, DE) est 12 cm.

9. Sonde selon la revendication 1, dans laquelle les canaux les plus petits (6, 8) sont séparés du canal le plus grand (4) à l'extrémité proximale de la sonde par une longueur de 25 cm et sont chacun pourvus d'un bouchon (22, 24) faisant partie intégrante de ceci.

10. Sonde selon la revendication 1, dans laquelle le canal le plus grand (4) est évasé (23) à son extrémité proximale (H).

11. Sonde selon la revendication 1, dans laquelle la portion de la sonde ayant les trous débouchants (10) la plus proche de la poche distale (BC) a une couleur différente sur une longueur de quelques centimètres.

12. Sonde selon la revendication 1, dans laquelle la section du canal le plus grand (4) à partir de la poche distale (BC) jusqu'au point d'extrémité (A) de la sonde est solide, a une longueur de 1 cm et est pourvue d'un trou débouchant (18) de quelques millimètres de diamètre.

13. Sonde selon la revendication 1, dans laquelle elle est pourvue d'une spirale (12) qui est enroulée autour d'une longueur (FE) de la sonde et est pourvue d'un anneau (14) auquel un poids (16) permettant une traction sous-cardiale fixe peut être suspendu.

14. Sonde selon la revendication 1, dans laquelle la poche proximale (DE) en état dégonflé est telle qu'elle permet à l'opérateur de faire glisser un endoscope dans le champ opératoire.
